(19) Europäisches Patentamt
European Patent Office
Office européen des brevets

(11) Veröffentlichungsnummer: **0 076 961**
**A1**

(12) # EUROPÄISCHE PATENTANMELDUNG

(21) Anmeldenummer: **82108907.5**

(22) Anmeldetag: **27.09.82**

(51) Int. Cl.³: **C 07 D 213/64**, A 01 N 53/00

(30) Priorität: **09.10.81 DE 3140092**

(43) Veröffentlichungstag der Anmeldung: **20.04.83**
**Patentblatt 83/16**

(84) Benannte Vertragsstaaten: **AT BE CH DE FR GB IT LI NL**

(71) Anmelder: **BAYER AG, Zentralbereich Patente, Marken und Lizenzen, D-5090 Leverkusen 1, Bayerwerk (DE)**

(72) Erfinder: **Fuchs, Rainer, Dr., Roeberstrasse 8, D-5600 Wuppertal 1 (DE)**
Erfinder: **Hammann, Ingeborg, Dr., Belfortstrasse 9, D-5000 Köln 1 (DE)**
Erfinder: **Behrenz, Wolfgang, Dr., Untergründemich 14, D-5063 Overath (DE)**
Erfinder: **Stendel, Wilhelm, Dr., In den Birken 55, D-5600 Wuppertal 1 (DE)**

(54) **Substituierte Vinylcyclopropancarbonsäure-(6-phenoxy-2-pyridinyl)-methylester, Verfahren zu ihrer Herstellung sowie ihre Verwendung als Schädlingsbekämpfungsmittel.**

(57) Die vorliegende Erfindung betrifft neue substituierte Vinylcyclopropancarbonsäure-(6-phenoxy-2-pyridinyl)-methylester der Formel (I)

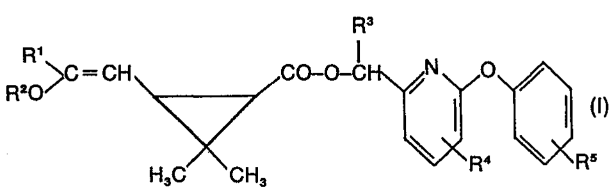

(I)

in welcher
$R^1$ für Fluoralkyl steht,
$R^2$ für gegebenenfalls substituierte Reste Alkyl, Cycloalkyl, Aralkyl oder Aryl steht und
$R^3$ für Wasserstoff, Cyano, Alkyl, Alkenyl oder Alkinyl steht, wobei letztere Reste bis zu 4 Kohlenstoffatome enthalten können und gegebenenfalls durch Halogen substituiert sind, sowie
$R^4$ und $R^5$ gleich oder verschieden sind und für Wasserstoff oder Halogen stehen.
Man erhält die neuen Verbindungen der Formel (I), wenn man Oxyalkenylcyclopropancarbonsäuren der Formel (II)

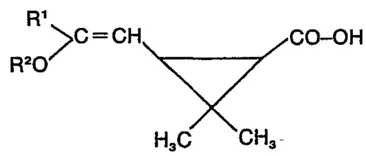

(II)

oder reaktionsfähige Derivate derselben, mit Phenoxypyridylalkoholen der Formel (III)

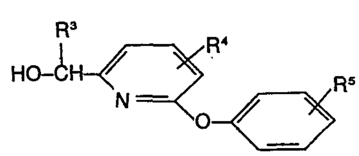

(III)

oder mit reaktionsfähigen Derivaten derselben umsetzt.
Sie zeichnen sich durch hohe pestizide, insbesondere insektizide und akarizide Wirkung aus.

EP 0 076 961 A1

BAYER AKTIENGESELLSCHAFT 5090 Leverkusen, Bayerwerk

Zentralbereich
Patente, Marken und Lizenzen Rt/Ma-c

# Substituierte Vinylcyclopropancarbonsäure-(6-phenoxy-2-pyridinyl)-methylester, Verfahren zu ihrer Herstellung sowie ihre Verwendung als Schädlingsbekämpfungsmittel

Die vorliegende Erfindung betrifft neue substituierte Vinylcyclopropancarbonsäure-(6-phenoxy-2-pyridinyl)-methylester, ein Verfahren zu ihrer Herstellung sowie ihre Verwendung als Schädlingsbekämpfungsmittel.

Es ist bekannt, daß bestimmte Vinylcyclopropancarbonsäureester, wie z.B. 3-(2-Methyl-propen-1-yl)-2,2-dimethylcyclopropan-1-carbonsäure-(3-phenoxy-benzyl)-ester, 3-(2,2-Dichlor-vinyl)-2,2-dimethyl-cyclopropan-1-carbonsäure-(3-phenoxy-benzyl)-ester und 3-(2,2-Dichlorvinyl)-2,2-dimethylcyclopropan-1-carbonsäure-α-cyano-(6-phenoxy-2-pyridinyl)-methylester insektizid und akarizid wirksam sind (vergleiche DE-OS 1 926 433, DE-OS 2 326 077 und DE-OS 2 810 881).

Die Wirkung dieser Verbindungen ist jedoch, insbesondere bei niedrigen Wirkstoffkonzentrationen und Aufwandmengen, nicht immer zufriedenstellend.

Es wurden nun neue substituierte Vinylcyclopropancarbonsäure-(6-phenoxy-2-pyridinyl)-methylester der Formel I

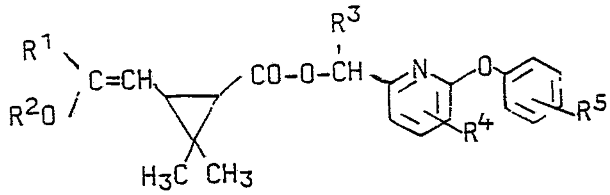

(I)

gefunden, in welcher

$R^1$ für Fluoralkyl steht,

$R^2$ für gegebenenfalls substituierte Reste Alkyl, Cycloalkyl, Aralkyl oder Aryl steht und

$R^3$ für Wasserstoff, Cyano, Alkyl, Alkenyl oder Alkinyl steht, wobei letztere Reste bis zu 4 Kohlenstoffatome enthalten können und gegebenenfalls durch Halogen substituiert sind, sowie

$R^4$ und $R^5$ gleich oder verschieden sind und für Wasserstoff oder Halogen stehen.

Die allgemeine Formel (I) schließt die verschiedenen möglichen Stereoisomeren und optischen Isomeren sowie deren Mischungen mit ein.

Man erhält die neuen Verbindungen der Formel (I), wenn man Oxyalkenylcyclopropancarbonsäuren der Formel II

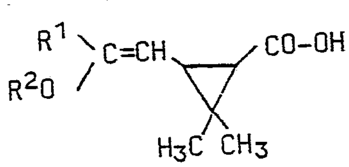

(II)

in welcher

$R^1$ und $R^2$ die oben angegebenen Bedeutungen haben,

oder reaktionsfähige Derivate derselben,

mit Phenoxypyridylalkoholen der Formel III

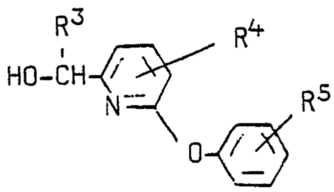

(III)

in welcher

$R^3$, $R^4$ und $R^5$ die oben angegebene Bedeutung haben,

oder mit reaktionsfähigen Derivaten derselben,

gegebenenfalls in Gegenwart von Säureakzeptoren, gegebenenfalls in Gegenwart von Katalysatoren und gegebenenfalls in Gegenwart von Verdünnungsmitteln umsetzt.

Die neuen substituierten Vinylcyclopropancarbonsäure-(6-phenoxy-2-pyridinyl)-methylester zeichnen sich durch hohe pestizide, insbesondere insektizide und akarizide Wirkung aus.

Überraschenderweise zeigen die erfindungsgemäßen Verbindungen der Formel I eine höhere insektizide und akarizide Wirkung als die aus dem Stand der Technik bekannten Verbindungen, wie die bereits erwähnten Vinylcyclopropancarbonsäureester: 3-(2-Methyl-propen-1-yl)-2,2-dimethyl-cyclopropan-1-carbonsäure-(3-phenoxy-benzyl)-ester, 3-(2,2-Dichlor-vinyl)-2,2-dimethylcyclopropan-1-carbonsäure-(3-phenoxy-benzyl)-ester und 3-(2,2-Dichlor-vinyl)-2,2-dimethylcyclopropan-1-carbonsäure-$\alpha$-cyano-(6-phenoxy-2-pyridinyl)-methylester.

Die Erfindung betrifft vorzugsweise Verbindungen der Formel (I), in welcher

$R^1$ für $C_1$-$C_3$-Fluoralkyl oder $C_1$-$C_3$-Chlor-fluoralkyl steht,

$R^2$ für gegebenenfalls substituierte Reste $C_1$-$C_4$-Alkyl, $C_5$-$C_6$-Cycloalkyl, Phenyl oder Benzyl steht, als Substituenten seien genannt Halogen insbesondere Fluor oder Chlor,

$R^3$ für Wasserstoff, Cyano, Methyl, Ethyl, Propyl, Ethenyl, Propenyl, Ethinyl oder Propinyl steht, sowie

$R^4$ und $R^5$ für Wasserstoff, Fluor, Chlor oder Brom stehen.

Die Erfindung betrifft insbesondere Verbindungen der Formel (I), in welcher

$R^1$ für Trifluormethyl steht,

$R^2$ für Methyl, Ethyl, iso-Propyl, n-Propyl, n-Butyl und iso-Butyl steht,

$R^3$ für Wasserstoff oder Cyano steht sowie

$R^4$ und $R^5$ für Wasserstoff stehen.

In einer bevorzugten Variante (a) des Herstellungsverfahrens für die Verbindungen der Formel (I) werden Oxyalkenylcyclopropan-carbonsäurechloride der Formel II a

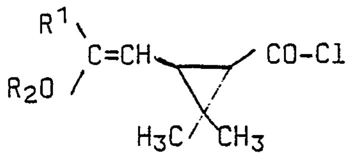

(II a)

in welcher

$R^1$ und $R^2$ die oben angegebenen Bedeutungen haben,

mit Phenoxypyridylalkoholen der Formel III (oben) in Gegenwart von Säureakzeptoren und unter Verwendung von Verdünnungsmitteln umgesetzt.

In einer weiteren bevorzugten Variante (b) - zur Herstellung von Verbindungen der Formel (I), in welcher $R^3$ für Cyano steht - werden die Verbindungen der Formel (II a) mit entsprechenden Phenoxypyridincarbaldehyden (Phenoxyformyl-pyridinen) der Formel IV

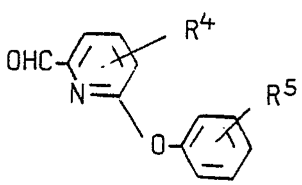

(IV)

in welcher

$R^4$ und $R^5$ die oben angegebenen Bedeutungen haben,

und wenigstens der äquimolaren Menge eines Alkalicyanids (Natrium- oder Kaliumcyanid) in Gegenwart von Wasser und eines mit Wasser nicht mischbaren organischen Lösungsmittels und gegebenenfalls in Gegenwart eines Katalysators umgesetzt.

Verwendet man als Ausgangsstoffe bei der Verfahrensvariante (a) beispielsweise 3-(2-Methoxy-3,3,3-trifluorpropen-1-yl)-2,2-dimethylcyclopropan-1-carbonsäurechlorid und 6-phenoxy-2-pyridyl-methanol, so kann die Reaktion durch folgendes Formelschema skizziert werden:

(a)

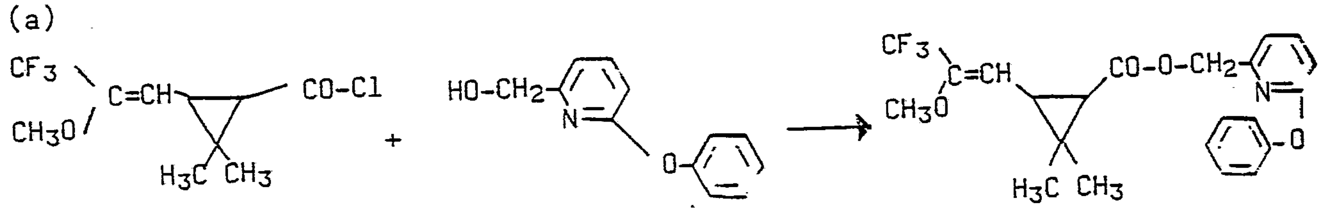

Verwendet man als Ausgangsstoffe bei der Verfahrensvariante (b) 3-(2-Ethoxy-3,3,3-trifluor-propen-1-yl)-2,2-dimethylcyclopropan-1-carbonsäurechlorid, Natriumcyanid und 6-Phenoxy-pyridin-2-carbaldehyd, so kann der Reaktionsverlauf durch folgendes Formelschema skizziert werden:

(b)

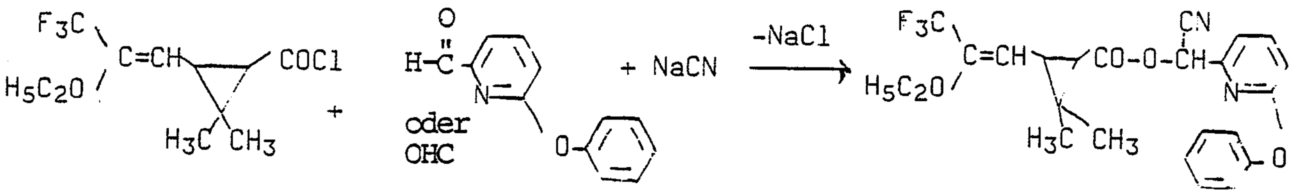


Die als Ausgangsstoffe zu verwendenden Oxyalkenylcyclopropancarbonsäuren sind durch Formel (II), die entsprechenden Säurechloride durch Formel (II a) definiert.

$R^1$ und $R^2$ haben darin vorzugsweise bzw. insbesondere die gleichen Bedeutungen, wie sie bei der Definition des entsprechenden Restes in Formel (I) vorzugsweise bzw. als insbesondere bevorzugt angegeben ist.

Als Beispiele für die Verbindungen der Formel (II) und die entsprechenden Säurechloride der Formel (II a) seien genannt:
3-(2-Methoxy-3,3,3-trifluor-propen-1-yl)-, 3-(2-Ethoxy-3,3,3-trifluor-propen-1-yl)-, 3-(2-n-Propoxy-3,3,3-trifluor-propen-1-yl)-, 3-(2-iso-Propoxy-3,3,3-trifluor-propen-1-yl)-, 3-(2-Methoxy-4,4,4,3,3-pentafluor-buten-1-yl)- und 3-(2-Ethoxy-5,5,5,4,4,3,3-heptafluor-penten-1-yl)-2,2-dimethyl-cyclopropan-1-carbonsäure sowie die entsprechenden Säurechloride.

Die Oxyalkenyl-cyclopropancarbonsäurechloride der Formel (II a) können nach üblichen Methoden aus den entsprechenden Carbonsäuren der Formel (II), durch Umsetzung mit einem Chlorierungsmittel wie z.B. Thionylchlorid, gegebenenfalls unter Verwendung eines Verdünnungsmittels wie z.B. Tetrachlorkohlenstoff, bei Temperaturen zwischen 10 und 100°C hergestellt werden.

Die Oxyalkenyl-cyclopropancarbonsäuren der Formel (II) sind bekannt (vergleiche DE-OS 2 919 820 (Le A 19 634)). Man erhält sie z.B. aus den entsprechenden Alkylestern durch mehrstündiges Erhitzen mit wäßrig-alkoholischer Alkalilauge wie z.B. Natronlauge, auf Temperaturen zwischen 50 und 150°C. Die Aufarbeitung erfolgt nach üblichen Methoden, beispielsweise durch Abdestillieren des Alkohols, Verdünnen mit Wasser, Ansäuern, Extrahieren mit Methylenchlorid, Trocknen der organischen Extrakte und Abdestillieren des Lösungsmittels.

Die weiter als Ausgangsstoffe zu verwendenden Phenoxypyridylalkohole sind durch Formel (III) definiert. Vorzugsweise bzw. insbesondere stehen darin $R^3$, $R^4$ und $R^5$ für diejenigen Reste, welche bereits bei der Definition von $R^3$, $R^4$ und $R^5$ in Formel (I) vorzugsweise bzw. als insbesondere bevorzugt genannt wurden.

Als Beispiele für die Ausgangsverbindungen der Formel (III) seien genannt:
6-Phenoxy-2-pyridylmethanol und 6-Phenoxy-2-pyridyl-α-cyano-methanol.

Die Verbindungen der Formel (III) sind bereits bekannt (vergleiche US-PS 4 163 787).

Die als Ausgangsstoffe verwendbaren Phenoxypyridincarbaldehyde sind durch Formel (IV) definiert. $R^4$ und $R^5$ haben darin die gleiche bevorzugte bzw. insbesondere Bedeutung wie die entsprechenden Reste in Formel (I).

Als Beispiel sei 6-Phenoxy-pyridin-2-carbaldehyd genannt.

Die Verbindungen der Formel (IV) sind ebenfalls bekannt (vergleiche US-PS 4 163 787).

Das Verfahren zu Herstellung der neuen Verbindungen der Formel (I) wird in allen Varianten vorzugsweise unter Verwendung von Verdünnungsmitteln durchgeführt. Als Verdünnungsmittel kommen praktisch alle inerten organischen Lösungsmittel in Frage. Hierzu gehören insbesondere aliphatische und aromatische, gegebenenfalls halogenierte Kohlenwasserstoffe, wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether, wie Diethyl- und Dibutylether, Glycoldimethylether und Diglycoldimethylether, Tetrahydrofuran und Dioxan, Ketone, wie Aceton, Methylethyl-, Methyl-iso-propyl- und Methyl-iso-butyl-keton, Ester wie Essigsäure-methylester und -ethylester, Nitrile, wie z.B. Acetonitril und Propionitril, Amide wie z.B. Dimethylformamid, Dimethylacetamid und N-Methyl-pyrrolidon, sowie Dimethylsulfoxid, Tetramethylensulfon und Hexamethylphosphorsäuretriamid.

Die Variante (a) des erfindungsgemäßen Verfahrens wird vorzugsweise in Gegenwart von Säureakzeptoren durchgeführt. Als Säureakzeptoren können die üblichen Säurebindemittel Verwendung finden. Besonders bewährt haben sich Alkalicarbonate und -alkoholate, wie Natrium- und

Kaliumcarbonat, Natrium- und Kaliumethylat bzw. -methylat, ferner aliphatische, aromatische oder heterocyclische Amine, beispielsweise Triethylamin, Trimethylamin, Dimethylanilin, Dimethylbenzylamin, Pyridin, Diazabicyclooctan, Diazabicyclononen und Diazabicycloundecen.

Die Variante (b) des erfindungsgemäßen Verfahrens wird in Gegenwart von Wasser und eines der oben genannten organischen Lösungsmittel, soweit mit Wasser nicht mischbar, durchgeführt. Hierfür sind insbesondere die oben genannten Kohlenwasserstoffe geeignet.

Als Katalysatoren werden bei der Verfahrensvariante (b) vorzugsweise Verbindungen verwendet, welche zum Transfer von Anionen aus Wasser in organische Lösungsmittel geeignet sind. Beispiele hierfür sind Benzyl-triethyl-ammoniumhydrogensulfat, Tetrabutylammonium-bromid und Methyl-trioctyl-ammonium-chlorid (Aliquat 336®).

In allen Verfahrensvarianten kann die Reaktionstemperatur innerhalb eines größeren Bereichs variiert werden. Im allgemeinen arbeitet man zwischen 0 und 100°C, vorzugsweise bei 10 bis 50°C.

Das erfindungsgemäße Verfahren wird im allgemeinen bei Normaldruck durchgeführt. Zur Durchführung des erfindungsgemäßen Verfahrens werden die Ausgangsstoffe gewöhnlich in äquimolaren Mengen eingesetzt. Ein Überschuß der einen oder anderen Reaktionskomponente bringt keine wesentlichen Vorteile. Die Ausgangsstoffe werden in geeigneten Verdünnungsmitteln zusammengegeben und, gegebenenfalls nach Zugabe eines Säureakzeptors und/oder eines Katalysators, bis zum Reaktionsende gerührt.

Die Aufarbeitung kann nach üblichen Methoden durchgeführt werden, beispielsweise indem man das Reaktionsgemisch gegebenenfalls mit Wasser und/oder einem mit Wasser nicht mischbaren organischen Lösungsmittel, wie z.B. Toluol, verdünnt, die organische Phase abtrennt, mit Wasser wäscht, trocknet, filtriert und vom Filtrat das Lösungsmittel unter vermindertem Druck und bei mäßig erhöhter Temperatur sorgfältig abdestilliert ('andestillieren').

Die neuen substituierten Vinylcyclopropancarbonsäure-(6-phenoxy-2-pyridinyl)-methylester zeichnen sich, wie bereits erwähnt, durch hohe insektizide und akarizide Wirksamkeit aus.

Sie können gegen pflanzenschädigende Insekten und Milben in Land- und Forstwirtschaft, im Vorratsschutz und Hygienebereich sowie gegen Ektoparasiten im veterinär-medizinischen Bereich eingesetzt werden.

Die Wirkstoffe eignen sich bei guter Pflanzenverträglichkeit und günstiger Warmblütertoxizität zur Bekämpfung von tierischen Schädlingen, insbesondere Insekten, Spinnentieren, die in der Landwirtschaft, in Forsten, im Vorrats- und Materialschutz sowie auf dem Hygienesektor vorkommen. Sie sind gegen normal sensible und resistente Arten

sowie gegen alle oder einzelne Entwicklungsstadien wirksam. Zu den oben erwähnten Schädlingen gehören:

Aus der Ordnung der Isopoda z.B. Oniscus asellus, Armadillidium vulgare, Porcellio scaber.
Aus der Ordnung der Diplopoda z.B. Blaniulus guttulatus.
Aus der Ordnung der Chilopoda z.B. Geophilus carpophagus Scutigera spec.
Aus der Ordnung der Symphyla z.B. Scutigerella immaculata.
Aus der Ordnung der Thysanura z.B. Lepisma saccharina.
Aus der Ordnung der Collembola z.B. Onychiurus armatus.
Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.
Aus der Ordnung der Dermaptera z.B. Forficula auricularia.
Aus der Ordnung der Isoptera z.B. Reticulitermes spp..
Aus der Ordnung der Anoplura z.B. Phylloxera vastatrix, Pemphigus spp., Pediculus humanus corporis, Haematopinus spp., Linognathus spp.
Aus der Ordnung der Mallophaga z.B. Trichodectes spp., Damalinea spp.
Aus der Ordnung der Thysanoptera z.B. Hercinothrips femoralis, Thrips tabaci.
Aus der Ordnung der Heteroptera z.B. Eurygaster spp., Dysdercus intermedius, Piesma quadrata, Cimex lectularius, Rhodnius prolixus, Triatoma spp.
Aus der Ordnung der Homoptera z.B. Aleurodes brassicae,

Bemisia tabaci, Trialeurodes vaporariorum, Aphis gossypii, Brevicoryne brassicae, Cryptomyzus ribis, Doralis fabae, Doralis pomi, Eriosoma lanigerum, Hyalopterus arundinis, Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp. Psylla spp.

Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp. Bucculatrix thurberiella, Phyllocnistis citrella, Agrotis spp., Euxoa spp., Feltia spp. Earias insulana, Heliothis spp., Laphygma exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Cacoecia podana, Capua reticulana, Choristoneura fumiferana, Clysia ambiguella, Homona magnanima, Tortrix viridana.

Aus der Ordnung der Coleoptera z.B. Anobium punctatum, Rhizopertha dominica, Bruchidius obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes

spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica.
Aus der Ordnung der Hymenoptera z.B. Diprion spp., Hoplocampa spp., Lasius spp., Monomorium pharaonis, Vespa spp.
Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.

Aus der Ordnung der Siphonaptera z.B. Xenopsylla cheopis, Ceratophyllus spp.
Aus der Ordnung der Arachnida z.B. Scorpio maurus, Latrodectus mactans.
Aus der Ordnung der Acarina z.B. Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp.

Die Wirstoffe können in die üblichen Formulierungen

übergeführt werden, wie Lösungen, Emulsionen, Suspensionen, Pulver, Schäume, Pasten, Granulate, Aerosole, Wirkstoffimprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen und ähnliches, sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chloräthylene oder Methylenchlorid, aliphatische Kohlenwasserstoffe, wie Cyclohexan oder Paraffine, z.B. Erdölfraktionen, Alkohole, wie Butanol oder Glycol sowie deren Äther und Ester, Ketone, wie Aceton, Methyläthylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur

und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen in Frage:
z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/ oder schaumerzeugende Mittel kommen in Frage: z.B. nichtionogene und anionische Emulgatoren, wie Polyoxyäthylen-Fettsäure-Ester, Polyoxyäthylen-Fettalkohol-Äther, z.B. Alkylarylpolyglykol-ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige und latexförmige Polymere verwendet werden, wie Gummiarabicum, Polyvinylalkohol, Polyvinylacetat.

Es können Farbstoffe wie anorganische Pigmente, z.B.

Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in ihren handelsüblichen Formulierungen sowie in den aus diesen Formulierungen bereiteten Anwendungsformen in Mischung mit anderen Wirkstoffen, wie Insektiziden, Lockstoffen, Sterilantien, Akariziden, Nematiziden, Fungiziden, wachstumsregulierenden Stoffen oder Herbiziden vorliegen. Zu den Insektiziden zählen beispielsweise Phosphorsäureester, Carbamate, Carbonsäureester, chlorierte Kohlenwasserstoffe, Phenylharnstoffe, durch Mikroorganismen hergestellte Stoffe und anderes.

Die erfindungsgemäßen Wirkstoffe können ferner in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muß.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 100 Gew.-% Wirkstoff, vorzugsweise zwischen 0,0001 und 1 Gew.-% liegen.

Die Anwendung geschieht in einer den Anwendungsformen angepaßten üblichen Weise.

Bei der Anwendung gegen Hygiene- und Vorratsschädlinge zeichnen sich die Wirkstoffe durch eine hervorragende Residualwirkung auf Holz und Ton sowie durch eine gute Alkalistabilität auf gekälkten Unterlagen aus.

Die erfindungsgemäßen Wirkstoffe eignen sich auch zur Bekämpfung von Ekto- und Endoparasiten auf dem veterinärmedizinischen Gebiet.

Die Anwendung der erfindungsgemäßen Wirkstoffe geschieht im Veterinärsektor in bekannter Weise, wie durch orale Anwendung in Form von beispielsweise Tabletten, Kapseln, Tränken, Granulaten, durch dermale Anwendung in Form beispielsweise des Tauchens (Dippen), Sprühens (Sprayen), Aufgießens (pour-on and spot-on) und des Einpuderns sowie durch parenterale Anwendung in Form beispielsweise der Injektion.

Herstellungsbeispiele

Beispiel 1

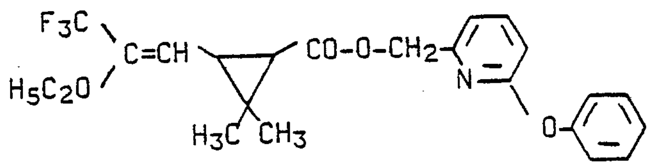

4,02 g (0,02 mol) (6-Phenoxy-2-pyridinyl)-methanol und 2 g Pyridin, werden in 100 ml wasserfreiem Toluol gelöst und bei 20 - 25°C 5,4 g (0,02 mol) 2,2-Dimethyl-3-(3,3,3-trifluor-2-ethoxy-propen-1-yl)-cyclopropancarbonsäurechlorid, gelöst in 20 ml wasserfreiem Toluol, unter Rühren zugetropft. Anschließend wird weitere 3 Stunden bei 25 - 35°C gerührt. Das Reaktionsgemisch wird in 150 ml Wasser gegossen, die organische Phase abgetrennt und nochmals mit 100 ml Wasser gewaschen. Anschließend wird die Toluolphase über Natriumsulfat getrocknet und das Lösungsmittel im Wasserstrahlvakuum abdestilliert. Letzte Lösungsmittelreste werden durch kurzes Andestillieren bei 60°C/1 Torr Badtemperatur entfernt. Man erhält 6,9 g (79,3 % der Theorie) 2,2-Dimethyl-3-(3,3,3-trifluor-2-ethoxy-propen-1-yl)-cyclopropancarbonsäure-(6-phenoxy-2-pyridinyl)-methylester als gelbes Öl mit dem Brechungsindex $n_D^{20}$ : 1,5092.

Beispiel 2

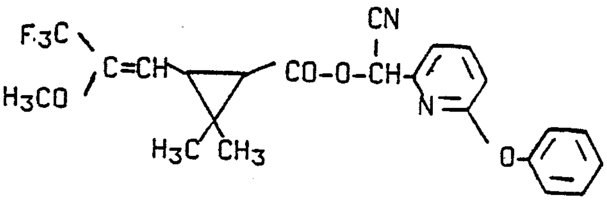

3,98 g (0,02 mol) 2-Formyl-6-phenoxy-pyridin und 5,13 g (0,02 mol) 2,2-Dimethyl-3-(3,3,3-trifluor-2-methoxy-propen-1-yl)-cyclopropancarbonsäurechlorid werden zusammen unter Rühren bei 20 - 25°C zu einer Mischung von 1,7 g Natriumcyanid, 2 ml Wasser, 100 ml Cyclohexan und 0,4 g Tetrabutylammoniumbromid getropft, und dann 4 Stunden bei 20 - 25°C gerührt. Anschließend wird die Reaktionsmischung mit 300 ml Toluol versetzt und 2 mal mit je 300 ml Wasser ausgeschüttelt. Die organische Phase wird abgetrennt, über Magnesiumsulfat getrocknet und das Lösungsmittel im Wasserstrahlvakuum abdestilliert. Letzte Lösungsmittelreste werden durch kurzes Andestillieren bei 60°C/1 Torr Badtemperatur entfernt. Man erhält 5,35 g (60 % der Theorie) 2,2-Dimethyl-3-(3,3,3-trifluor-methyl-2-methoxy-propen-1-yl)-cyclopropancarbonsäure-α-cyano-(6-phenoxy-2-pyridinyl)-methylester als zähes Öl. Die Struktur wird durch das $^1$H-NMR-Spektrum bewiesen.

$^1$H-NMR in $CDCl_3$/TMS $\tau$ (ppm):

| | | |
|---|---|---|
| aromatische H | : | 2,14 - 3,21 (m/8 H) |
| Benzyl-H | : | 3,69 (s(l.c.) /1 H) |
| Vinyl-H | : | 3,95 - 4,14 und 4,6 - 4,76 (2 x d/1 H) |
| Methoxy-H | : | 6,19 - 6,3 (m/3 H) |
| Cyclopropyl-H | : | 7,47 - 8,36 (m/2 H) |
| Dimethyl-H | : | 8,6 - 8,83 (m/6 H). |

Analog den Beispielen 1 und 2 können noch folgende Beispiele hergestellt werden:

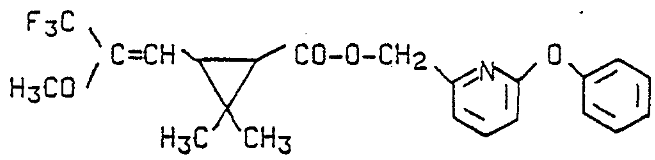

Brechungsindex $n_D^{20}$: 1,5142
Ausbeute: 82,3 % d.Th.

(3)

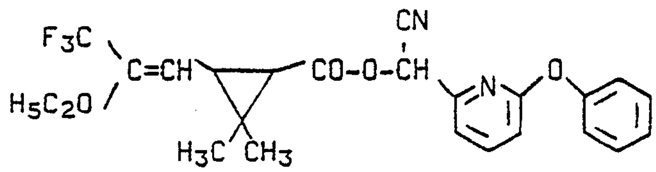

(4)

Ausbeute: 71% d.Th.

$^1$H-NMR in $CDCl_3$/TMS τ (ppm):

| | | | |
|---|---|---|---|
| Benzyl-H | : | 3,71 | (S(l.c.)/1 H) |
| Ethoxy-H | : | 5,88 - 6,24 | (m/2 H) |
| | | 8,55 - 8,88 | (m/3 H) |

Verwendungsbeispiele

In den nachfolgenden Beispielen werden die nachstehend angegebenen Verbindungen als Vergleichssubstanzen eingesetzt:

(A) 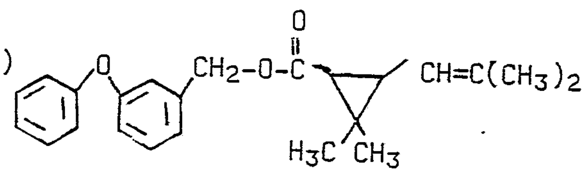

(B) 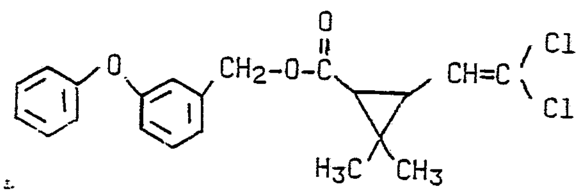

(C) 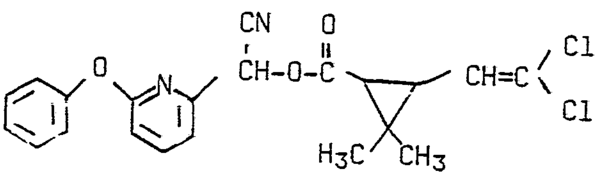

Beispiel A

Myzus-Test

Lösungsmittel: 3 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea), die stark von der Pfirsichblattlaus (Myzus persicae) befallen sind, werden durch Tauchen in die Wirkstoffzubereitung der gewünschten Konzentration behandelt.

Nach der gewünschten Zeit wird die Abtötung in % bestimmt. Dabei bedeutet 100 %, daß alle Blattläuse abgetötet wurden; 0 % bedeutet, daß keine Blattläuse abgetötet wurden.

Bei diesem Test zeigen z.B. die folgenden Verbindungen der Herstellungsbeispiele überlegene Wirksamkeit gegenüber dem Stand der Technik:
1, 2, 3 und 4.

Beispiel B

Tetranychus-Test (resistent)

Lösungsmittel: 3 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Bohnenpflanzen (Phaseolus vulgaris), die stark von allen Entwicklungsstadien der gemeinen Spinnmilbe oder Bohnenspinnmilbe (Tetranychus urticae) befallen sind, werden durch Tauchen in die Wirkstoffzubereitung der gewünschten Konzentration behandelt.

Nach der gewünschten Zeit wird die Abtötung in % bestimmt. Dabei bedeutet 100 %, daß alle Spinnmilben abgetötet wurden; 0 % bedeutet, daß keine Spinnmilben abgetötet wurden.

Bei diesem Test zeigen z.B. die folgenden Verbindungen der Herstellungsbeispiele überlegene Wirksamkeit gegenüber dem Stand der Technik:
1, 2, 3 und 4.

Beispiel C

$LT_{100}$-Test für Dipteren

Testtiere : Aedes aegypti
Lösungsmittel : Aceton

2 Gewichtsteile Wirkstoff werden in 1000 Volumenteilen Lösungsmittel aufgenommen. Die so erhaltene Lösung wird mit weiterem Lösungsmittel auf die gewünschte geringeren Konzentrationen verdünnt.

2,5 ml Wirkstofflösung werden in eine Petrischale pipettiert. Auf dem Boden der Petrischale befindet sich ein Filterpapier mit einem Durchmesser von etwa 9,5 cm. Die Petrischale bleibt so lange offen stehen, bis das Lösungsmittel vollständig verdunstet ist. Je nach Konzentration der Wirkstofflösung ist die Menge Wirkstoff pro $m^2$ Filterpapier verschieden hoch. Anschließend gibt man etwa 25 Testtiere in die Petrischale und bedeckt sie mit einem Glasdeckel.

Der Zustand der Testtiere wird laufend kontrolliert. Es wird diejenige Zeit ermittelt, welche für einen 100 %igen knock-down-Effekt notwendig ist.

Bei diesem Test zeigen z.B. die folgenden Verbindungen der Herstellungsbeispiele überlegene Wirkung gegenüber dem Stand der Technik:
1, 2, 3 und 4.

0076961

Beispiel D

Test mit Boophilus microplus resistent

Lösungsmittel: 35 Gewichtsteile Ethylenglykolmonomethylether
35 Gewichtsteile Nonylphenolpropylglykolether

Zur Herstellung einer zweckmäßigen Wirkstoffzubereitung vermischt man drei Gewichtsteile Wirkstoff mit sieben Gewichtsteilen des oben angegebenen Lösungsmittel-Gemisches und verdünnt das so erhaltene Konzentrat mit Wasser auf die gewünschte Konzentration.

10 adulte Boophilus micropuls res. werden in die zu testende Wirkstoffzubereitung 1 Minute getaucht. Nach Überführung in Plastikbecher und Aufbewahrung in einem klimatisierten Raum wird der Abtötungsgrad bestimmt.

Bei diesem Test zeigen z.B. die folgenden Verbindungen der Herstellungsbeispiele überlegene Wirkung gegenüber dem Stand der Technik:
1, 2, 3 und 4.

Beispiel E

Test mit Lucilia cuprina res.-Larven

| | |
|---|---|
| Lösungsmittel | 35 Gewichtsteile Ethylenglykolmonomethylether |
| | 35 Gewichtsteile Nonylphenolpolyglykolether |

Zur Herstellung einer zweckmäßigen Wirkstoffzubereitung vermischt man drei Gewichtsteile Wirkstoff mit sieben Gewichtsteilen des oben angegebenen Lösungsmittel-Gemisches und verdünnnt das so erhaltene Konzentrat mit Wasser auf die jeweils gewünschte Konzentration.

Etwa 20 Lucilia cuprina res.-Larven werden in ein Teströhrchen gebracht, welches ca. 1 $cm^2$ Pferdefleisch und 0,5 ml der Wirkstoffzubereitung enthält. Nach 24 Stunden wird der Abtötungsgrad bestimmt.

Bei diesem Test zeigen z.B. die folgenden Verbindungen der Herstellungsbeispiele überlegene Wirkung gegenüber dem Stand der Technik: (1), (2), (3) und (4).

Patentansprüche

1. Vinylcyclopropancarbonsäure-(6-phenoxy-2-pyridinyl)-methylester der Formel I

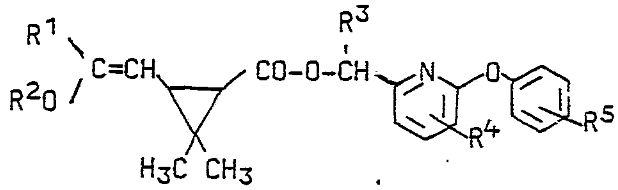

(I)

in welcher

$R^1$ für Fluoralkyl steht,

$R^2$ für gegebenenfalls substituierte Reste Alkyl, Cycloalkyl, Aralkyl oder Aryl steht und

$R^3$ für Wasserstoff, Cyano, Alkyl, Alkenyl oder Alkinyl steht, wobei letztere Reste bis zu 4 Kohlenstoffatome enthalten können und gegebenenfalls durch Halogen substituiert sind, sowie

$R^4$ und $R^5$ gleich oder verschieden sind und für Wasserstoff oder Halogen stehen.

2. Verfahren zur Herstellung der Vinylcyclopropancarbonsäure-(6-phenoxy-2-pyridinyl)-methylester der Formel I

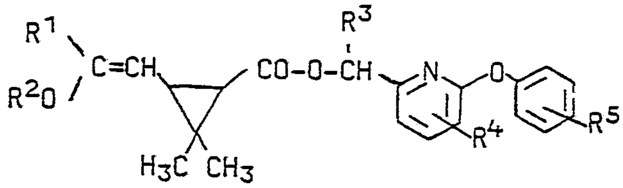

(I)

in welcher

$R^1$ für Fluoralkyl steht,

$R^2$ für gegebenenfalls substituierte Reste Alkyl, Cycloalkyl, Aralkyl oder Aryl steht und

$R^3$ für Wasserstoff, Cyano, Alkyl, Alkenyl oder Alkinyl steht, wobei letztere Reste bis zu 4 Kohlenstoffatomen enthalten können und gegebenenfalls durch Halogen substituiert sind, sowie

$R^4$ und $R^5$ gleich oder verschieden sind und für Wasserstoff oder Halogen stehen,

dadurch gekennzeichnet, daß man Oxyalkenylcyclopropancarbonsäuren der Formel II

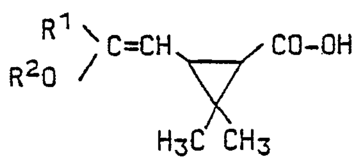

(II)

in welcher

$R^1$ und $R^2$ die oben angegebenen Bedeutungen haben,

oder reaktionsfähige Derivate derselben, mit Phenoxypyridylalkoholen der Formel III

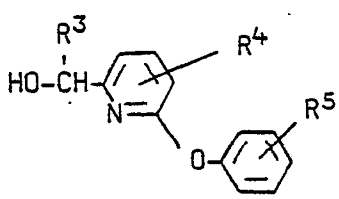

(III)

in welcher

$R^3$, $R^4$ und $R^5$ die oben angegebene Bedeutung haben, oder mit reaktionsfähigen Derivaten derselben,

gegebenenfalls in Gegenwart von Säureakzeptoren, gegebenenfalls in Gegenwart von Katalysatoren und gegebenenalls in Gegenwart von Verdünnungsmitteln umsetzt.

3. Verbindungen gemäß Anspruch 1 der Formel (I), in welcher

$R^1$ für $C_1$-$C_3$-Fluoralkyl oder $C_1$-$C_3$-Chlor-fluoralkyl steht,

$R^2$ für gegebenenfalls substituierte Reste $C_1$-$C_4$-Alkyl, $C_5$-$C_6$-Cycloalkyl, Phenyl oder Benzyl steht, welche gegebenenfalls durch Halogen substituiert sind,

$R^3$ für Wasserstoff, Cyano, Methyl, Ethyl, Propyl, Ethenyl, Propenyl, Ethinyl oder Propinyl steht, sowie

$R^4$ und $R^5$ für Wasserstoff, Fluor, Chlor oder Brom stehen.

4. Verbindungen gemäß Anspruch 1 der Formel (I), in welcher

$R^1$ für Trifluormethyl steht,

$R^2$ für Methyl, Ethyl, iso-Propyl, n-Propyl, n-Butyl und iso-Butyl steht,

$R^3$ für Wasserstoff oder Cyano steht sowie

$R^4$ und $R^5$ für Wasserstoff stehen.

5. Schädlingsbekämpfungsmittel, gekennzeichnet durch einen Gehalt an mindestens einem substituierten Vinylcyclopropancarbonsäure-(6-phenoxy-2-pyridinyl)-methylester der Formel (I).

6. Verwendung von substituierten Vinylcyclopropancarbonsäure-(6-phenoxy-2-pyridinyl)-methylester der Formel (I) zur Bekämpfung von Schädlingen.

7. Verfahren zur Bekämpfung von Schädlingen, dadurch gekennzeichnet, daß man substituierten Vinylcyclopropancarbonsäure-(6-phenoxy-2-pyridinyl)-methylester der Formel (I) auf Schädlinge und/oder ihren Lebensraum einwirken läßt.

8. Verfahren zur Herstellung von Schädlingsbekämpfungsmitteln, dadurch gekennzeichnet, daß man substituierten Vinylcyclopropancarbonsäure-(6-phenoxy-2-pyridinyl)-methylester der Formel (I) mit Streckmitteln und/oder oberflächenaktiven Mitteln vermischt.

Europäisches Patentamt

**EUROPÄISCHER RECHERCHENBERICHT**

0076961

Nummer der Anmeldung

EP 82 10 8907

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. ³) |
|---|---|---|---|
| | --- | | C 07 D 213/64<br>A 01 N 53/00 |
| A | EP-A-0 020 954 (BAYER)<br>* Ansprüche * | 1-8 | |
| | --- | | |
| A | FR-A-2 383 927 (DOW CHEMICAL)<br>* Ansprüche * | 1-8 | |
| | ----- | | |
| | | | RECHERCHIERTE SACHGEBIETE (Int. Cl. ³) |
| | | | C 07 D 213/00 |

Der vorliegende Recherchenbericht wurde für alle Patentansprüche erstellt.

| Recherchenort | Abschlußdatum der Recherche | Prüfer |
|---|---|---|
| DEN HAAG | 14-12-1982 | WRIGHT M.W. |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument